Europäisches Patentamt

European Patent Office

Office européen des brevets

(19)

(11) Veröffentlichungsnummer: **0 208 144**
**B1**

(12) **EUROPÄISCHE PATENTSCHRIFT**

(45) Veröffentlichungstag der Patentschrift :
20.09.89

(21) Anmeldenummer : 86107700.6

(22) Anmeldetag : 06.06.86

(51) Int. Cl.$^4$: **A 61 K 9/16**, A 61 K 9/22,
A 61 K 31/135

(54) Ambroxol oder Bromhexin enthaltendes Arzneimittelgranulat.

(30) Priorität : 04.07.85 DE 3524003

(43) Veröffentlichungstag der Anmeldung :
14.01.87 Patentblatt 87/03

(45) Bekanntmachung des Hinweises auf die Patenterteilung : 20.09.89 Patentblatt 89/38

(84) Benannte Vertragsstaaten :
AT BE CH DE FR GB IT LI LU NL SE

(56) Entgegenhaltungen :
EP--A-- 0 014 514
EP--A-- 0 069 259
WO--A--84/046 74
DE--A-- 1 927 130
DE--A-- 2 412 960

(73) Patentinhaber : HEUMANN PHARMA GMBH & CO
18-28 Heideloffstrasse
D-8500 Nürnberg (DE)

(72) Erfinder : Grätzel von Grätz, Jochen, Dr. Dipl.-Chem.
Ulmenstrasse 29
D-8501 Feucht (DE)
Erfinder : Prochazka, Josef
Ludwigstrasse 13
D-8438 Berg b. Neumarkt/Opf. (DE)

(74) Vertreter : Kraus, Walter, Dr. et al
Patentanwälte Kraus, Weisert & Partner Thomas-Wimmer-Ring 15
D-8000 München 22 (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Ambroxol oder Bromhexin oder deren Salze enthaltendes Arzneimittelgranulat mit verzögerter Wirkstofffreisetzung zur oralen Anwendung, üblicherweise als Retardformen bekannt, und ein Verfahren zu seiner Herstellung. Die erfindungsgemäßen Granulate werden zu pharmazeutischen Zubereitungen verarbeitet, die eine kontrollierte Wirkstoffabgabe in der Form aufweisen, daß nach Freisetzung einer Initialdosis über einen Zeitraum von 7 und mehr Stunden der Wirkstoff anhaltend gleichmäßig und vollständig abgegeben wird.

Zur Steuerung der Wirkstofffreisetzung aus pharmazeutischen Darreichungsformen sind zahlreiche Vorschläge gemacht worden und eine Fülle von Verfahren beschrieben. Einen Überblick gibt P. Speiser in H. Sucker, P. Fuchs und P. Speiser, Pharmazeutische Technologie, Georg Thieme Verlag Stuttgart, 1978, S. 486-498.

Für orale Anwendung sind zusammengefaßt im wesentlichen zwei Verfahren in unterschiedlichsten Varianten gebräuchlich :

a) die Freisetzung ist membrangesteuert, d. h. die Abgabe des Wirkstoffs aus der Darreichungsform wird durch gezielte Auflösung oder Permeabilität eines aufgebrachten Films gesteuert,

b) die Freisetzung ist matrixgesteuert, d. h. der Wirkstoff liegt fein verteilt in einer Matrix vor und wird entweder durch gesteuerte Erosion oder durch gezielt eingestellte Porosität des sich nicht auflösenden Formlings verfügbar.

Beide genannten Verfahren sind für übliche feste Darreichungsformen wie Tabletten, Kapseln, Granulate und überzogene Tabletten in Verwendung.

Als Hilfsstoffe zur Herstellung der genannten verzögert freisetzenden Darreichungsformen sind einerseits natürliche synthetische oder halbsynthetische Filmbildner im Gebrauch oder aber Gerüststoffe wie Lipide, Kunststoffe oder Quellstoffe.

Nach den genannten Methoden bereitet es normalerweise keine Schwierigkeiten, Retardzubereitungen herzustellen mit Wirkstoffen, die im Magen und Darmsaft gleichermaßen $p_H$-unabhängig löslich sind. Es gibt allerdings eine Reihe von Wirkstoffen, deren Löslichkeit bei niedrigem $p_H$ des Magens gut, bei höherem $p_H$ des Dünndarms aber äußerst schlecht ist.

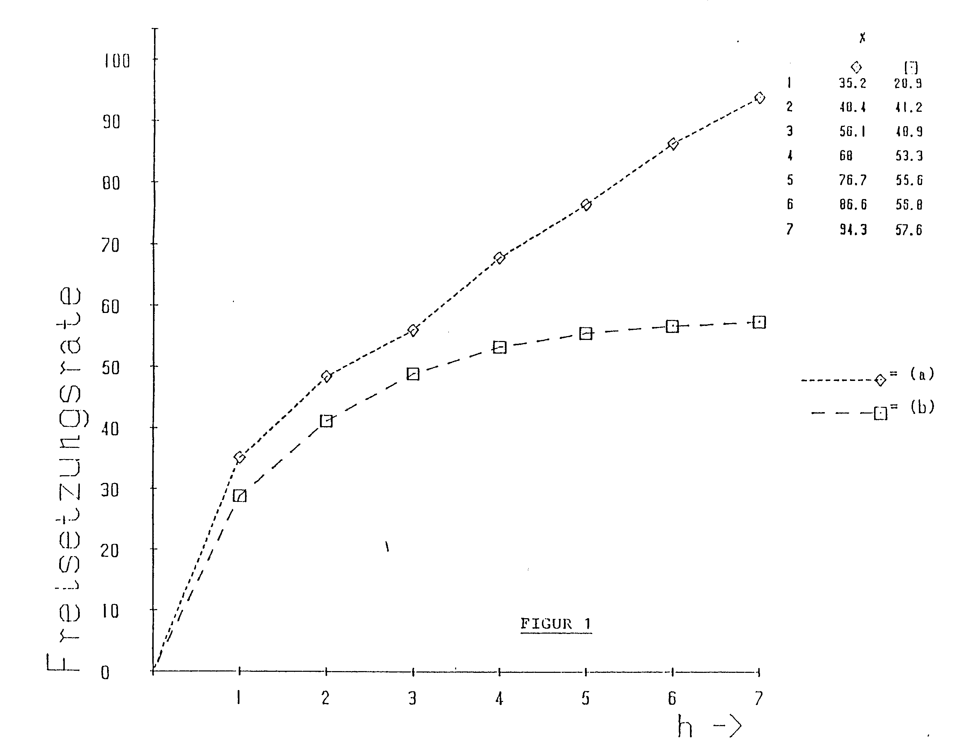

In Fig. 1 ist die Freisetzung eines $p_H$-unabhängig löslichen Wirkstoffes (a) dem eines im Magensaft löslichen, im Darmsaft schwer löslichen Wirkstoffs (b) gegenübergestellt. Während in Figur 1a die Abgabe gleichmäßig und vollständig innerhalb von 7 Stunden verläuft, wird der Wirkstoff im Beispiel 1b nur in saurem $p_H$ herausgelöst, im Bereich des Dünndarm-$p_{Hs}$ erfolgt keine ausreichende Freisetzung.

Ein ähnliches Beispiel für eine Matrix-Tablette mit Bromhexin ist in der DE-A-3 126 703 beschrieben.

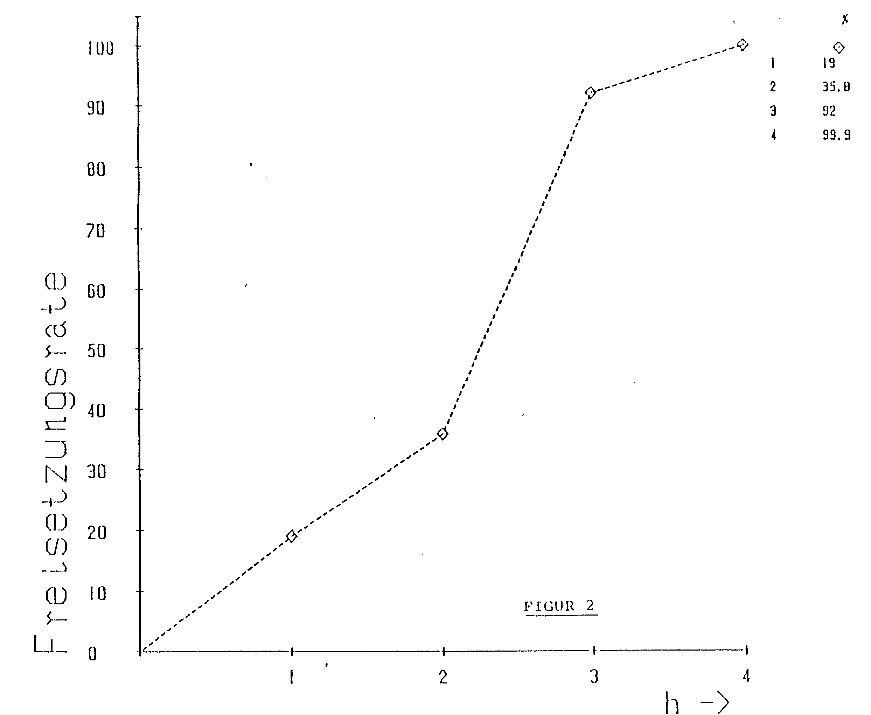

Fig. 2 zeigt die Freisetzungskurve eines $p_H$-abhängig löslichen Wirkstoffs in einem membrangesteuerten System. Es handelt sich um Ambroxolhydrochlorid in mit einem gebräuchlichen $p_H$-abhängig löslichen Filmbildner überzogenen Pellets. Die Freisetzung erfolgt hier ungleichmäßig und in nicht erwünschter Weise schnell.

Es hat in der Literatur nicht an Versuchen gefehlt, das Problem der $p_H$-abhängigen Löslichkeit in Retardformen zu bewältigen. In der bereits genannten DE-A-3 126 703 werden Bromhexin-Retardformen beschrieben, welche aus einem mit einem Überzug versehenen Teilchen bestehen, wobei der Starterkern aus dem genußsäurehaltigen Wirkstoff besteht, der von einem Hüllenanteil umgeben ist.

In der europäischen Patentanmeldung EP-A-0 040 439 wird eine Arzneimittelform mit verzögerter Freigabe beschrieben, welche

a) 10 bis 50 Gew.-% gesättigte Fettsäuren und/oder ihre Metallsalze und

b) einen pharmakologisch aktiven Bestandteil sowie

c) übliche Füllstoffe, Verdünnungsmittel, Stabilisatoren und/oder Bindemittel und andere Hilfsstoffe enthält.

Gemäß Seite 5 der Beschreibung sind Beispiele für die langkettigen Fettsäuren Stearinsäure und Palmitinsäure. Beispiele für Zusatzstoffe sind gemäß Seite 7, letzter Absatz und Seite 8, erster Absatz enterische Lacke wie Schellack, Celluloseacetatphthalat (CAP), Hydroxypropylmethylcellulosephthalat (HPMCP) und Copolymere der Methacrylsäure. Diese Zusatzstoffe werden jedoch zur Beschichtung verwendet und auf die Oberfläche der Körnchen aus Wirkstoff aufgetragen.

In der DE-C-2 439 538 wird ein Verfahren zur Herstellung von oral zu verabreichenden Arzneimitteln mit verzögerter Wirkstofffreigabe beschrieben, das dadurch gekennzeichnet ist, daß man einen Wirkstoff mit einer bestimmten Korngröße, ein Bindemittel mit einer bestimmten Korngröße und gegebenenfalls einen Füllstoff zu Granulaten verpreßt.

Weiterhin ist es üblich, verschieden überzogene Granulate oder Preßlinge in einer Darreichungsform, z. B. einer Kapsel, zu vereinen. Diese Verfahren haben den Nachteil, daß sie nur sehr umständlich zu realisieren sind, nicht für alle Wirkstoffe geeignet sind bzw. ungleichmäßige, stufenweise Freisetzungsprofile liefern.

In der DE-A-1 927 130 wird ein Depotarzneimittel in Tablettenform beschrieben, welches neben dem Wirkstoff ein hydrophobes, die Lösung verzögerndes Mittel, ein die Freisetzung bewirkendes, in Säure unlösliches Mittel und ein in Wasser lösliches oder dispergierbares Bindemittel enthält. Dieses bekannte Depotarzneimittel in Tablettenform enthält das die Freisetzung bewirkende säureunlösliche Mittel in einer Menge im Bereich von 0,1 bis 5,0 Gew.-% und es muß immer ein Bindemittel enthalten. Eine gezielte Steuerung der Freisetzung des Arzneimittels ist bei dieser Depotform nicht möglich. Stellt man beispielsweise ein Ambroxol enthaltendes Arzneimittel gemäß den Angaben der DE-A-1 927 130 her, so beträgt die Freisetzung von Ambroxol nach 8 Stunden weniger als 70 %. Ein solches Arzneimittel ist für die Praxis völlig ungeeignet.

In der WO-A-8 404 674 wird ein Arzneimittelgranulat mit verzögerter Wirkstofffreisetzung und ein Verfahren zu seiner Herstellung beschrieben. Dieses bekannte Arzneimittelgranulat enthält ein hydrophobes Kohlenhydrat-Polymeres, welches als Bindemittel für die anderen Bestandteile dient und als Voraussetzung für eine Direktverpressung unter Umgehung von Schmelz- und Lösungsvorgängen dient. Es ist nicht löslich und dient deshalb nicht zur Steuerung der Freisetzung des Wirkstoffs, insbesondere nicht pH-abhängig löslich und kann daher zur Steuerung der Freisetzung von Wirkstoffen, deren Löslichkeit stark pH-abhängig ist, nicht verwendet werden. Die auf Seite 5, Zeile 4 ff der genannten Literaturstelle aufgeführten Polymeren sind im Darmsaft nicht löslich. Die bekannten Granulate werden lediglich durch Kompression hergestellt. Schmelz- und Extrusionsgranulate lassen sich nicht nach diesem bekannten Verfahren wie auf Seite 2, Zeile 14 und Seite 3, Zeile 10 angegeben wird, herstellen.

Auf Seite 6 werden in dieser Literaturstelle zahlreiche Arzneimittel beschrieben, die nach dem bekannten Verfahren zu Arzneimitteln mit verzögerter Wirkstofffreisetzung verarbeitet werden können. Die in der Liste aufgeführten biologisch aktiven Substanzen haben sehr unterschiedliche Löslichkeitsprofile. Ambroxol oder Bromhexin, die im Magensaft löslich und im Darmsaft schwer löslich sind, lassen sich nach diesem Verfahren nicht zu Retardformen verarbeiten.

In der europäischen Patentanmeldung EP-A-69 259 wird eine neue Bromhexin-Retardform und ein Verfahren zu seiner Herstellung beschrieben. Gemäß dem in dieser europäischen Patentschrift beschriebenen Verfahren werden die derzeit im Handel befindlichen Retardformen von Bromhexin hergestellt. Diese bekannten Retardformen bestehen aus einem mit einem Überzug versehenen Teilchen oder Tabletten und sind umständlich und kompliziert herzustellen, da die Dicke der verschiedenen Überzüge genau kontrolliert werden muß.

Es handelt sich hierbei um eine sehr aufwendige, teure und schwer reproduzierbare Verfahrensweise mit niedriger Produktausbeute.

Der vorliegenden Erfindung liegt die Aufgabe zugrunde, ein Arzneimittelgranulat mit verzögerter Wirkstofffreisetzung auf den in Magensaft löslichen in Darmsaft schwer löslichen Wirkstoffen Ambroxol oder Bromhexin zur Verfügung zu stellen, wobei die Freisetzung gleichmäßig und kontrolliert erfolgt und das Granulat auf einfache Weise hergestellt werden kann.

Es wurde überraschenderweise gefunden, daß ein Arzneimittelgranulat, welches die Wirkstoffe Ambroxol oder Bromhexin, ein Lipid und einen Filmbildner in bestimmten Mengen enthält, die Forderung nach gleichmäßiger pH-unabhängiger Freisetzung von an sich stark pH-abhängigen löslichen Wirkstoffen erfüllt.

Gegenstand der Erfindung ist ein Arzneimittelgranulat mit verzögerter Wirkstofffreisetzung aus den in Magensaft löslichen, in Darmsaft schwer löslichen Wirkstoffen Ambroxol oder Bromhexin und einem Filmbildner, dadurch gekennzeichnet, daß es

a) 1 Gew.-Teil Ambroxol oder Bromhexin oder deren Salze,

b) 0,2 bis 10 Gew.-Teile eines niedrigschmelzenden Lipids mit einem Schmelzbereich von 40 bis 90 °C aus der Gruppe Fettalkohol mit mehr als 13 Kohlenstoffatomen, eine höhere Fettsäure, ein Glycerid, Mono-, Di- oder Triester von Glycerin mit Palmitin- oder Stearinsäure oder pulverisierte natürliche oder synthetische Wachse,

c) 0,2 bis 10 Gew.-Teile eines in Magensaft unlöslichen, in Darmsaft löslichen Filmbildners in Pulverform,

d) gegebenenfalls weitere übliche Formulierungshilfsmittel enthält, wobei alle Bestandteile als inniges Gemisch vorliegen und wobei die Komponenten a) und b) als alleinige Binde- und Retardierungsmittel vorliegen.

Die Erfindung betrifft weiterhin ein Verfahren zur Herstellung eines Ambroxol oder Bromhexin enthaltenden Arzneimittelgranulats zur oralen Anwendung mit verzögerter Wirkstofffreisetzung, daß dadurch gekennzeichnet ist, daß man

a) 1 Gew.-Teil Ambroxol oder Bromhexin oder deren Salze,

b) 0,2 bis 10 Gew.-Teile eines niedrigschmelzenden Lipids mit einem Schmelzbereich von 40 bis 90 °C aus der Gruppe Fettalkohol mit mehr als 13 Kohlenstoffatomen, eine höhere Fettsäure, ein Glycerid, Mono-, Di- oder Triester von Glycerin mit Palmitin- oder Stearinsäure oder pulverisierte natürliche oder synthetische Wachse,

c) 0,2 bis 10 Gew.-Teile eines in Magensaft unlöslichen, in Darmsaft löslichen Filmbildners in Pulverform,

d) gegebenenfalls weitere übliche Formulierungshilfsmittel ohne den Zusatz anderer Binde- und Retardierungsmittel außer den Komponenten b) und c) vermischt und zu Schmelz- bzw. Extrusionsgranulaten verarbeitet.

Die Herstellung derartiger Retardgranulate erfolgt in an sich bekannter Weise durch z. B. Sprüherstarrung geschmolzener Suspensionen in der angegebenen Zusammensetzung, durch Agglomeration in Gegenwart von Wärme in einem geeigneten Mischer oder durch Extrusion der Pulvermischung durch Düsenplättchen oder Lochwalzen.

Es ist als ausgesprochen überraschend zu bezeichnen, daß Granulate der angegebenen Zusammensetzung eine gleichmäßige pH-unabhängige Freisetzung aufweisen. Zwar ist dem Fachmann bekannt, daß die Porosität einer lipophilen Matrix durch lösliche Zuschlagstoffe wie anorganische Salze, Kohlenhydrate, höhere Alkohole oder Genußsäuren erhöht werden kann, jedoch führt dies bei stark pH-abhängig löslichen Wirk-

stoffen zu unerwünscht hohen Anfangsfreisetzungen, wenn gleichzeitig gewährleistet sein soll, daß die Substanz in 8 Stunden vollständig freigesetzt wird. Weiterhin ist bekannt, daß Magensaft unlösliche, Darmsaft lösliche Filmbildner als Matrixkomponenten eingesetzt werden können. Deren komplette Auflösung beim $p_H$ des Dünndarms bewirkt aber lediglich einen Zerfall der Matrix und eine zwar verzögerte, aber unerwünscht stufenweise Freisetzung.

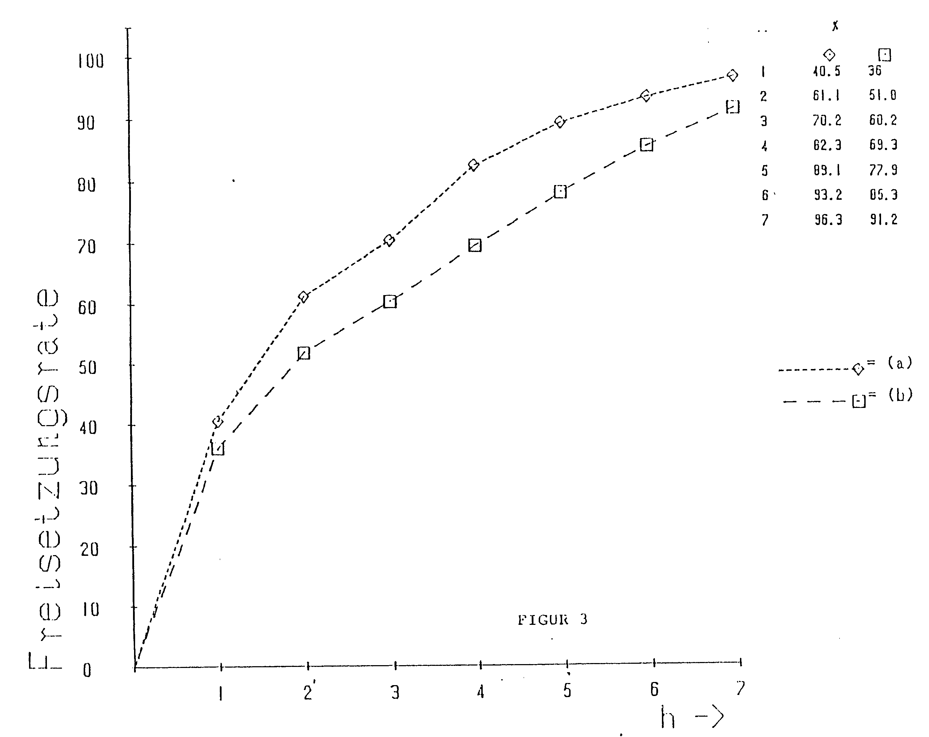

Die erfindungsgemäße Kombination in den angegebenen Mengenverhältnissen bewirkt einerseits eine für Matrixretardformen übliche und erwünschte Anfangsfreisetzung bei niedrigem $p_H$, die nicht zu hoch ist, andererseits bei steigendem $p_H$ eine allmählich zunehmende Porosität, die auch die Diffusion von bei steigendem $p_H$ schwerer löslichen Wirkstoff ermöglicht (vgl. Fig. 3).

Es war überraschend und nicht vorherzusehen, daß es ein Mischungsverhältnis Lipid zu darmsaftlöslichem Lackpulver gibt, bei dem im üblichen Untersuchungszeitraum von 8 Stunden bei insgesamt ausreichender Anfangsfreisetzung lediglich die Porosität der Matrix ansteigt, diese aber nicht zerfällt. Weiterhin überraschend erwies sich, daß die erfindungsgemäß zusammengesetzte Matrix eine außerordentliche Stabilität aufweist.

Einerseits ist bekannt, daß sich infolge von Kristallisationsvorgängen innerhalb einer plastifizierten oder aus einer Schmelze erstarrten Lipidphase das Freisetzungsverhalten des inkorporierten Wirkstoffs bei Lagerung verändert, in der Regel verlangsamt.

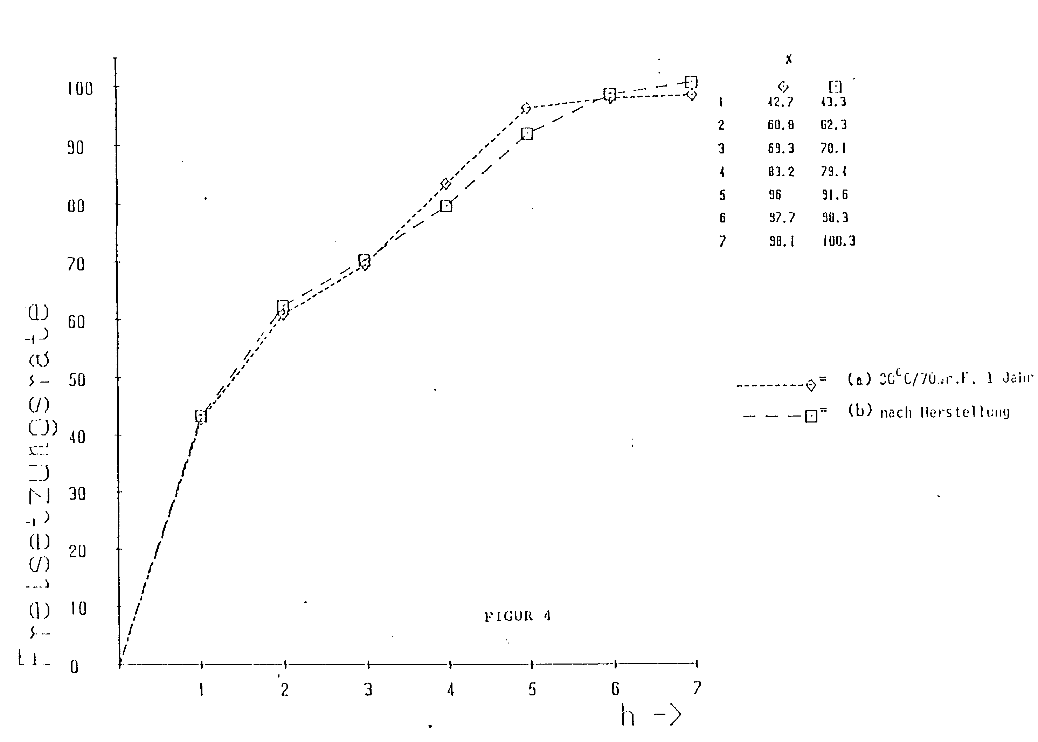

Andererseits ist jedem Fachmann gegenwärtig, daß magensaftresistente Lacke, insbesondere Celluloseacetatphthalat (CAP) und Hydroxypropylmethylcellulosephthalat (HPMCP), dazu neigen, sich unter Einfluß von feuchter Wärme hydrolytisch zu spalten. Die Folge sind ebenfalls starke Veränderungen im Freigabeverhalten nach Lagerung hier im Sinne einer Beschleunigung. So ist es auch für den Fachmann als äußerst überraschend zu bezeichnen, daß Granulate in der erfindungsgemäßen Zusammensetzung selbst nach Lagerung in feuchter Wärme (30 °C, 70 % rel. Feuchte, Fig. 4a) keine signifikante Änderung der Wirkstofffreisetzung gegenüber dem Ausgangswert (Fig. 4b) erfahren.

In dem erfindungsgemäßen Arzneimittelgranulat werden 0,2 bis 10 Gewichtsteile eines niedrig schmelzenden Lipides verwendet. Vorzugsweise werden 0,5 bis 5 Gewichtsteile verwendet. Das Lipid besitzt einen Schmelzbereich von 40 bis 90 °C, vorzugsweise von 55 bis 75 °C.

Die erfindungsgemäß verwendeten Lipide sind ebenfalls bekannt. Geeignet sind z. B. Fettalkohole mit mehr als 13, insbesondere 16-20, C-Atomen, wie Cetyl- oder Stearylalkohol, höhere Fettsäuren, wie Stearinsäure, Glyceride insbesondere hydrierte pflanzliche Öle, wie hydriertes Baumwollsamen- oder Rizinusöl, ferner Mono-, Di- oder Triester von Glycerin mit Palmitin- oder Stearinsäure, pulverisierte natürliche oder synthetische Wachse, wie Bienen- oder Carnaubawachs, oder Montanwachse oder deren Mischungen.

Die erfindungsgemäß verwendeten, in Magensaft unlöslichen, in Darmsaft löslichen Filmbildner in Pulverform sind gleichfalls bekannt. Geeignet sind Schellack, Celluloseacetatphthalat, Hydroxypropylmethylcellulosephthalat bzw. Polyacrylsäurederivate. Diese werden in einer Menge von 0,2 bis 10, vorzugsweise 0,5 bis 5, Gewichtsteile verwendet und im Gegensatz zu bekannten Granulaten bzw. Verfahren werden diese nicht als Filmbildner, d. h. zum Überziehen der Teilchen aus Wirkstoff und Lipid eingesetzt, sondern sie werden mit dem Wirkstoff und dem Lipid vermischt.

Als weitere Formulierungshilfsmittel lassen sich Fließregulierungsmittel, wie hochdisperse Kieselsäure, Füllstoffe, wie Lactose oder Cellulose, oder Farbstoffe, z. B. fein gemahlene Eisenoxise, verwenden.

Die Herstellung der erfindungsgemäßen Formulierung erfolgt in an sich bekannter Weise durch Mischen der pulverförmigen Rohstoffe und anschließender Granulation in einem doppelwandigen erwärmten Mischer oder Friktionsmischer oder aber durch Sprüherstarrung einer geschmolzenen Suspension. Weiterhin geeignet sind Extrusionsverfahren unter Verwendung von Granulierwalzen, Zahnradgranulierwalzen, Schneckenextrudern oder Tablettiermaschinen.

Die entstehenden Granulate oder Stränge werden mittels geeigneter Mühlen oder Siebe auf die erforderliche Teilchengröße gebracht und unter Zusatz üblicher Hilfsmittel tablettiert bzw. ohne Zusätze in Hartgelatinekapseln abgefüllt.

Es zeigen

Fig. 1 : Freisetzung eines $p_H$-unabhängig löslichen Wirkstoffes (a) und eines $p_H$-abhängig löslichen Wirkstoffes (b) aus einer lipophilen Matrix.

Fig. 2 : Freisetzung eines in Darmsaft schwer löslichen Wirkstoffes aus Pellets mit darmsaftlöslichem Überzug.

Fig. 3 : Freisetzung aus einem Ambroxol HCL-Granulat, das erfindungsgemäß hergestellt wurde (a) CAP, (b) HPMCP.

Fig. 4 : Freisetzung eines erfindungsgemäßen Granulats in Abhängigkeit der Lagerbedingungen.
a) 30 °C/70 % rel. Feuchte 1 Jahr
b) nach Herstellung

Die folgenden Ausführungsbeispiele erläutern die Erfindung :

Beispiel 1

75,0 g Ambroxolhydrochlorid, fein gepulvert, werden mit 90,0 g Celluloseacetatphthalatpulver und 70,0 g Montanwachspulver vermischt. Die Mischung wird durch Lochscheiben mit einem Lochdurchmesser von 1,0 mm gepreßt. Die resultierenden Stränge werden zerkleinert, abgesiebt und in einer Dosis von 235,0 mg in Hartgelatinekapseln abgefüllt.

Beispiel 2

6 kg Bromhexinhydrochlorid, 10 kg Methacryl-säure- Methacrylsäureester- Mischpolymerisat-pulver, 10 kg Stearinsäure und 0,1 kg Eisenoxid gelb werden gemischt und mittels einer Lochwalzengranuliermaschine zu Granulaten verarbeitet. Diese werden in einer Dosis von 262,0 mg in Hartgelatinekapseln abgefüllt.

## Patentansprüche

1. Arzneimittelgranulat mit verzögerter Wirkstofffreisetzung aus den in Magensaft löslichen, in Darmsaft schwer löslichen Wirkstoffen Ambroxol oder Bromhexin und einem Filmbildner, dadurch gekennzeichnet, daß es

a) 1 Gew.-Teil Ambroxol oder Bromhexin oder deren Salze,

b) 0,2 bis 10 Gew.-Teile eines niedrigschmelzenden Lipids mit einem Schmelzbereich von 40 bis 90 °C aus der Gruppe Fettalkohol mit mehr als 13 Kohlenstoffatomen, eine höhere Fettsäure, ein Glycerid, Mono-, Di- oder Triester von Glycerin mit Palmitin- oder Stearinsäure oder pulverisierte natürliche oder synthetische Wachse,

c) 0,2 bis 10 Gew.-Teile eines in Magensaft unlöslichen, in Darmsaft löslichen Filmbildners in Pulverform,

d) gegebenenfalls weitere übliche Formulierungshilfsmittel enthält, wobei alle Bestandteile als inniges Gemisch vorliegen und wobei die Komponenten b) und c) als alleinige Binde- und Retardierungsmittel vorliegen.

2. Verfahren zur Herstellung eines Ambroxol oder Bromhexin enthaltenden Arzneimittelgranulats zur oralen Anwendung mit verzögerter Wirkstofffreisetzung, nach Anspruch 1 dadurch gekennzeichnet, daß man

a) 1 Gew.-Teil Ambroxol oder Bromhexin oder deren Salze,

b) 0,2 bis 10 Gew.-Teile eines niedrigschmelzenden Lipids mit einem Schmelzbereich von 40 bis 90 °C aus der Gruppe Fettalkohol mit mehr als 13 Kohlenstoffatomen, eine höhere Fettsäure, ein Glycerid, Mono-, Di- oder Triester von Glycerin mit Palmitin- oder Stearinsäure oder pulverisierte natürliche oder synthetische Wachse,

c) 0,2 bis 10 Gew.-Teile eines in Magensaft unlöslichen, in Darmsaft löslichen Filmbildners in Pulverform,

d) gegebenenfalls weitere· übliche Formulierungshilfsmittel ohne den Zusatz anderer Binde- und Retardierungsmittel außer den Komponenten b) und c) vermischt und zu Schmelz- bzw. Extrusionsgranulaten verarbeitet.

## Claims

1. Pharmaceutical granulate with delayed release of active ingredient, composed of the active ingredient ambroxol or bromohexine, which are soluble in the gastric juice and difficultly soluble in the intestinal juice, and a film forming agent, characterised in that it contains

a) 1 part by weight of ambroxol or bromohexine or salts thereof,

b) from 0.2 to 10 parts by weight of a low melting lipid having a melting range of from 40 to 90 °C from the group comprising fatty alcohols containing more than 13 carbon atoms, a higher fatty acid, a glyceride, mono-, di- or triesters of glycerol with palmitic or stearic acid and pulverulent, natural or synthetic waxes,

c) from 0.2 to 10 parts by weight of a film forming agent in powder form which is insoluble in the gastric juice and soluble in the intestinal juice, and

d) optionally other conventional formulating auxiliaries,

all the components being present as an intimate mixture and components b) and c) being present as the only binding and retarding agents.

2. A process for the preparation of a pharmaceutical granulate containing ambroxol or bromohexine for oral administration with delayed release of active ingredient according to Claim 1, characterised in that

a) 1 part by weight of ambroxol or bromohexine or salts thereof,

b) from 0-2 to 10 parts by weight of a low melting lipid having a melting range of from 40 to 90 °C from the group comprising fatty alcohols containing more than 13 carbon atoms, a higher fatty acid, a glyceride, a mono-, di-, or triester of glycerol with palmitic or stearic acid and pulverised natural or synthetic waxes,

c) from 0.2 to 10 parts by weight of a film forming agent in powder form which is insoluble in the gastric juice and soluble in the intestinal juice, and

d) optionally other conventional formulating auxiliaries are mixed together without the addition of binders and retarding agents other than components b) and c) and worked up into melt granulates or extrusion granulates.

## Revendications

1. Granulé pharmaceutique à libération de substance active retardée, comprenant de l'ambroxol ou de la bromhexine comme substances actives solubles dans le suc gastrique et peu solubles dans le suc intestinal, et un agent filmogène, caractérisé en ce qu'il contient

a) 1 partie en poids d'ambroxol ou de bromhexine ou de leurs sels,

b) 0,2 à 10 parties en poids d'un lipide à bas point de fusion ayant un intervalle de fusion de 40 °C à 90 °C, appartenant au groupe constitué par un alcool gras à plus de 13 atomes de carbone, un acide gras supérieur, un glycéride, les mono-, di- ou triesters de la glycérine avec l'acide palmitique ou stéarique ou une cire naturelle ou synthétique pulvérisée,

c) 0,2 à 10 parties en poids d'un agent filmogène, insoluble dans le suc gastrique et soluble dans le suc intestinal, sous forme de poudre,

d) éventuellement d'autres adjuvants de formulation courants, tous les constituants se trouvant en mélange intime et les constituants b) et c) étant présents comme seuls liants et agents de retardement.

2. Procédé pour la préparation d'un granulé pharmaceutique contenant de l'ambroxol ou de la bromhexine pour administration orale avec libération de substance active retardée, selon la revendication 1, caractérisé en ce qu'on mélange

a) 1 partie en poids d'ambroxol ou de bromhexine ou de leurs sels,

b) 0,2 à 10 parties en poids d'un lipide à bas point de fusion ayant un intervalle de fusion de 40 °C à 90 °C, appartenant au groupe constitué par un alcool gras à plus de 13 atomes de carbone, un acide gras supérieur, un glycéride, les mono-, di- ou triesters de la glycérine avec l'acide palmitique ou stéarique ou une cire naturelle ou synthétique pulvérisée,

c) 0,2 à 10 parties en poids d'un agent filmogène, insoluble dans le suc gastrique et soluble dans le suc intestinal, sous forme de poudre,

d) éventuellement d'autres adjuvants de formulation courants sans l'addition d'autres liants et agents de retardement en dehors des constituants b) et c),

et on traite ce mélange de manière à le transformer en granulés par fusion ou par extrusion.

Freisetzungsrate

0 10 20 30 40 50 60 70 80 90 100

h →

1 2 3 4 5 6 7

FIGUR 1

| x | [ ] |
|---|---|
| 1 | 35.2 | 20.9 |
| 2 | 40.4 | 41.2 |
| 3 | 50.1 | 40.9 |
| 4 | 68 | 53.3 |
| 5 | 76.7 | 55.6 |
| 6 | 86.6 | 55.8 |
| 7 | 94.3 | 57.6 |

◇ = (a)
□ = (b)

1

Freisetzungsrate

FIGUR 2

h →

| | ◇ x |
|---|---|
| 1 | 19 |
| 2 | 35.8 |
| 3 | 92 |
| 4 | 99.9 |

FIGUR 3

**Freisetzungsrate (%)**

FIGUR 4

h →

| x | ◇ | □ |
|---|-----|------|
| 1 | 42.7 | 43.3 |
| 2 | 60.8 | 62.3 |
| 3 | 69.3 | 70.1 |
| 4 | 83.2 | 79.4 |
| 5 | 96 | 91.6 |
| 6 | 97.7 | 98.3 |
| 7 | 98.1 | 100.3 |

- - - - ◇ = (a) 30°C/70%r.f., 1 Jahr

— — □ = (b) nach Herstellung

4